# EUROPEAN PATENT APPLICATION

(11) **EP 1 454 622 A1**
(43) Date of publication of application: **08.09.2004**
(21) Application number: 04251232.7
(22) Date of filing: 03.03.2004
(51) Int. Cl.: A61K 7/48

(54) **Skin moisturizing composition**

(30) Priority: 04.03.2003 US 379075
(71) Applicant: Johnson & Johnson Consumer Companies, Inc., Skillman, New Jersey 08558-9418 (US)
(72) Inventor: Lukenbach, Elvin R,, Flemington, NJ 08822 (US); Nystrand, Glenn A., Lebanon, NJ 08833 (US); Walsh, Star Marie, Upper Black Eddy, PA 18972 (US)
(74) Representative: Mercer, Christopher Paul

(57) **Abstract**

A composition and method of use thereof to moisturize skin, the composition having the formula:
an alkoxylated fatty alcohol acid ester of Formula I;

   R₁-O-[CH₂-CH(CH₃)-O]_{y}-[(CH₂)₂-O]ₓ-C(O)-CH₂-B-CH₂-C(O)-[O-(CH₂)₂]ₓ-(O-CH(CH₃)-CH₂]-O-R₂ (I)

   wherein:
   B is alkylene of 0 to 10 carbon atoms which may be saturated or unsaturated, substituted or unsubstituted, or R₃-O-[CH₂-CH(CH₃)-O]_{y}-[(CH₂)₂-O]ₓ- C(O)-C(OH)<;and
   R_{1,} R₂ and R₃ may be the same or different and each are fatty alcohols of 10 to 22 carbons which may be saturated or unsaturated, substituted or unsubstituted, x is 0 to 40 and y is 0 to 30 with the proviso that the total of all x^{s} and y^{s} is at least 6; and
at least one emollient, wherein the emollient is not mineral oil or petrolatum.

## Description

### Background of the Invention

### Field of the Invention

The present invention relates to a composition that is useful for moisturizing skin. More specifically, this invention is related to such compositions that provide water retention in the skin without leaving a greasy feel on the surface of the skin. The compositions are free of mineral oil or petrolatum.

### Description of the Prior Art

It is well known that mineral oil and petrolatum are emollients. Mineral oil and petrolatum are derived from petroleum. Both materials are hydrophobic and have a greasy feel. Mineral oil and petrolatum provide excellent moisturization to skin where they are applied. They are typically applied to the skin in the form of creams or lotions.

Creams or lotions that contain mineral oil or petrolatum also typically contain surfactants to enable the formation of stable oil in water emulsions. Although the creams and lotions containing mineral oil or petrolatum perform very well as moisturizers, they tend to leave the skin feeling greasy. We have found that many consumers want a composition that moisturizes like mineral oil or petrolatum, but does not leave the skin feeling greasy.

United States Patent Nos. 5,302,377, 5,455,025, 5,597,555, and 6,476,254 relate to emollient compositions for topical application. The patents describe fatty alkoxylate esters of aliphatic and aromatic dicarboxylic and tricarboxylic acids. The esters are taught as being useful as emollients. The patents also teach that the esters may be combined with, or used in place of, mineral oil or petrolatum.

Natural emollients, such as oils derived from plants are well known in the art. It is generally understood that such materials do not moisturize the skin as well as mineral oil or petrolatum.

Despite the disclosure of the prior art, there is a continuing need for a composition that provides skin moisturization without leaving the skin feeling greasy.

### Summary of the Invention

The present invention provides a composition comprising an alkoxylated fatty alcohol acid ester of Formula I;

R₁-O-[CH₂-CH(CH₃)-O]_{y}-[(CH₂)₂-O]ₓ-C(O)-CH₂-B-CH₂-C(O)-[O-(CH₂)₂]ₓ-[O-CH(CH₃)-CH₂]-O-R₂ (I)

wherein:
B is alkylene of 0 to 10 carbon atoms which may be saturated or unsaturated, substituted or unsubstituted, or R₃-O-[CH₂-CH(CH₃)-O]_{y}-[(CH₂)₂-O]ₓ- C(O)-C(OH)<;and
R₁, R₂ and R₃ may be the same or different and each are fatty alcohols of 10 to 22 carbons which may be saturated or unsaturated, substituted or unsubstituted, x is 0 to 40 and y is 0 to 30 with the proviso that the total of all x^{s} and y^{s} is at least 6; and at least one emollient,
wherein the emollient is not mineral oil or petrolatum.
Also provided in accordance with the present invention is a method of moisturizing skin comprising applying to the skin, in an amount effective to moisturize said skin, a composition comprising: an alkoxylated fatty alcohol acid ester of Formula I as defined above, with the proviso that the total of all x^{s} and y^{s} is at least 6; and at least one emollient,
wherein the emollient is not mineral oil or petrolatum.

### Detailed Description of Preferred Embodiments

The compositions of the present invention may be provided in various forms, such as gels, creams, lotions, cleansers, and the like. The formulations may be solutions, oil in water emulsions, water in oil emulsions and the like.

The first component of the compositions of the present invention is the alkoxylated fatty alcohol acid ester of Formula I described above. In a preferred compound, x is 0, y is 3, B is 4, and R₁ and R₂ are each 14 carbons long and, in particular, myristyl alcohol. This compound is known as Di-polypropylene glycol-3 myristyl ether adipate, and is commercially available through Croda, Inc. as CROMOLLIENT DP3A. The ester may provide various functions for compositions of the present invention, including use as a film forming agent.

The amount of alkoxylated fatty alcohol acid ester of Formula I utilized in the compositions of the present invention may vary, depending on the type of formulation desired. For water in oil emulsions, the amount of ester may range from about 60% to about 90% by weight, based on the total weight of the composition. For oil in water emulsions, the amount of ester may range from about 5% to about 40% by weight, based on the total weight of the composition. For a solution, the amount of ester may range from about 1% to about 95% by weight, based on the total weight of the composition.

The second component of the compositions of the present invention is an emollient that is not mineral oil or petrolatum. Suitable emollients include, but are not limited to, polypropylene glycol ("PPG")-15 stearyl ether, PPG-10 cetyl ether, PPG-4 lauryl ether, vitamin E acetate, polyethyleneglycol ("PEG")-7 glyceryl cocoate, lanolin, lauroyl lysine, almond oil, sesame seed oil, oat kernel oil, rapeseed oil, macadamia nut oil, soybean oil, polydimethylsiloxane, and combinations thereof. A combination of sesame seed oil and polydimethylsiloxane is preferred.

The amount of emollient in the compositions of the present invention may vary depending on the type of formulation desired. Typically, the amount of emollient may range from about 3% to about 15%, preferably from about 5% to about 10% by weight, based on the total weight of the composition. For a water in oil emulsion, the amount of emollient may range from about 5% to about 40% in oil emulsion by weight, based on the total weight of the composition. For a solution, the amount of emollient may range from about 5% to about 99% by weight, based on the total weight of the composition.

The compositions of the present invention may include surfactants. Anionic, nonionic, cationic, amphoteric surfactants, and combinations thereof may be useful. Suitable nonionic surfactants include, but are not limited to, alkyl glycosides, such as octyl glucoside, decyl glucoside, and lauryl glucoside; ethylene oxide/propylene oxide copolymers; polyoxyethylene derivatives of polyol esters; and sucrose esters. When utilized, the amount of nonionic surfactant in the compositions of the present invention may range from about 0.1% to about 20%, preferably from about 0.5% to about 10%, and more preferably from about 0.5% to about 5% by weight, based on the total weight of the composition.

Betaine surfactants may also be useful in the compositions of the present invention. As used herein, betaines are derived from alkyl amidopropyl dimethylamine. They can exist in only two forms: cationic at low pH and isoelectric at intermediate pH. Suitable betaine surfactants include, but are not limited to, alkyl betaines, amidoalkyl betaines, phosphobetaines, pyrophosphobetaines, and mixtures thereof. When utilized, the amount of betaine surfactant in the compositions of the present invention may range from about 0.1% to about 20%, preferably from about 0.5% to about 10%, and more preferably from about 0.5% to about 5% by weight, based on the total weight of the composition.

Amphoteric surfactants may be useful in the compositions of the present invention. As used herein, amphoteric surfactants are derivatives of alkyl hydroxyethyl imidazolines formed through a reaction with sodium chloroacetate. They are true amphoterics in that they exist in three different forms depending on pH: cationic at low pH, zwitterionic at intermediate pH, and anionic at high pH. Suitable amphoteric surfactants include, but are not limited to, amphocarboxylates, amidoalkyl sultaines, amphophosphates, carboxyalkyl alkyl polyamines, and mixtures thereof. When utilized, the amount of amphoteric surfactant in the compositions of the present invention may range from about 0.1 % to about 20%, preferably from about 0.5% to about 10%, and more preferably from about 0.5% to about 5% by weight, based on the total weight of the composition.

Anionic surfactants may also be utilized in the compositions of the present invention. Suitable anionic surfactants include, but are not limited to sodium lauryl sulfate, sodium laureth sulfate, sodium salt of fatty acids, cetyl phosphate, taurates, acylamino acids, and lactylates. When utilized, the amount of anionic surfactant in the compositions of the present invention may range from about 0.1% to about 20%, preferably from about 0.5% to about 10%, and more preferably from about 0.5% to about 5% by weight.

When the compositions of the present invention are emulsions, emulsifiers may be used. Suitable emulsifiers include, but are not limited to: ethoxylated alcohols; glycertl stearate; sorbitan esters, ethoxylated sorbitan esters, ethoxylated and propoxylated alkyl glucosides, polyglyceryl esters, silicone copolyols, distearyldimonium chloride, and polyethylene glycol stearate.

Emulsion stabilizers may also be useful. Suitable emulsion stabilizers include, but are not limited to, cetyl alcohol and acrylates/C10-30 alkyl acrylate crosspolymer. The amount of emulsion stabilizer may range from about 0.1% to about 2% by weight, based on the total weight of the composition.

The compositions of the present invention may also contain water. The amount of water utilized will vary, depending on the type of formulation desired. Generally, the amount of water may range from 0 to 95% by weight, based on the total weight of the composition.

The compositions of the present invention may also include one or more optional ingredients nonexclusively including a thickening agent, occlusive agents, secondary conditioners, humectants, chelating agents, and additives which enhance their appearance, feel and fragrance, such as colorants, fragrances, preservatives, pH adjusting agents, and the like.

Commercially available thickening agents that are capable of imparting the appropriate viscosity to the compositions are suitable for use in this invention. If used, the thickener should be present in the compositions in an amount sufficient to raise the Brookfield viscosity of the composition to the desired viscosity. Examples of suitable thickening agents nonexclusively include: mono or diesters of 1) polyethylene glycol of formula: HO-(CH₂CH₂O)_{z}H, wherein z is an integer from about 3 to about 200; and 2) fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; xanthan gum; magnesium aluminum silicate; and mixtures thereof. Preferred thickeners include xanthan gum and magnesium aluminum silicate.

Occlusive agents are known in the art. A preferred occlusive agent is polydecene. When utilized in the present invention, the amount of occlusive agent may range from about 0.5% to about 10% by weight, based on the total weight of the composition.

Commercially available humectants, which are capable of providing moisturization and conditioning properties to the composition, are suitable for use in the present invention. The humectant may be present in an amount of from about 0 percent to about 10 percent, preferably from about 0.5 percent to about 5 percent, and more preferably from about 0.5 percent to about 3 percent, based on the overall weight of the composition. Examples of suitable humectants nonexclusively include: 1) water soluble liquid polyols selected from the group comprising glycerine, propylene glycol, hexylene glycol, butylene glycol, dipropylene glycol, and mixtures thereof; 2) polyalkylene glycol of the formula: HO-(R"O)_{b}-H, wherein R" is an alkylene group having from about 2 to about 3 carbon atoms and b is an integer of from about 2 to about 10; 3) polyethylene glycol ether of methyl glucose of formula CH₃-C₆H₁₀O₅-(OCH₂CH₂)_{c}-OH, wherein c is an integer from about 5 to about 25; 4) urea; and 5) mixtures thereof, with glycerine being the preferred humectant.

Examples of suitable chelating agents include those which are capable of protecting and preserving the compositions of this invention. Preferably, the chelating agent is EDTA, and more preferably is tetrasodium EDTA, available commercially from Dow Chemical Company of Midland, Michigan under the tradename, "Versene 100XL" and is present in an amount, based upon the total weight of the composition, from about 0 to about 0.5 percent, and preferably from about 0.01 percent to about 0.10 percent.

Suitable preservatives include, but are not limited to, Quaternium-15, available commercially as "Dowicil 200" from the Dow Chemical Corporation of Midland, Michigan, methylparaben, propylparaben, ethylparaben, diazolidinyl urea, and benzalkonium chloride and are present in the composition in an amount, based upon the total weight of the composition, from about 0 to about 2 percent, and preferably from about 0.05 percent to about 1 percent.

The pH of the compositions of this invention is preferably maintained in the range of from about 3 to about 7.5, and more preferably from about 5.5 to about 7.0. The compositions of the present invention may be useful for delivering active ingredients to the skin. Suitable active ingredients include, but are not limited to, alpha hydroxy acids, antiseptic agents, and sunscreen agents.

The above described composition may be prepared by combining the desired components in a suitable container and mixing them under ambient conditions by any conventional mixing means known in the art, such as via mechanically stirred propeller, paddle, and the like. Although the order of mixing is not critical, it is preferable to pre-blend certain components, such as the fragrance and the nonionic surfactant before adding such components into the main mixture.

Several examples are set forth below to further illustrate the nature of the invention and the manner of carrying it out. However, the invention should not be considered as being limited to the details thereof.

### Examples: Preparation of Moisturizing Compositions

The materials for three oil in water emulsion formulations are listed in Tables 1, 2 and 3:

**Table 1**

| Sample 1 - Ingredients | Amount |
|---|---|
| Water | 82.00 |
| Acrylates / C10-30 alkyl acrylate cross polymer) | 0.25 |
| Magnesium aluminum silicate | 0.40 |
| Xanthan gum | 0.30 |
| Colloidal Oat Flour | 0.25 |
| Glycerin | 2.0 |
| EDTA | 0.05 |
| Sesame seed oil | 6.0 |
| CROMOLLIENT DP3A | 5.0 |
| RHODORSIL 5193 | 0.20 |
| Glyceryl stearate / PEG 100 stearate | 1.25 |
| Cetyl alcohol | 1.25 |
| Butylated hydroxytoluene | 0.02 |
| Almond Oil | 0.01 |
| Oat kernel oil | 0.01 |
| Methylparaben | 0.20 |
| Ethylparaben | 0.15 |
| Propylparaben | 0.10 |
| Sodium Hydroxide (20% solution) | 0.10 |
| Fragrance | 0.15 |
| Diazolidinyl urea | 0.30 |

The thickening agents and oat flour were dispersed in the water. The batch was headed to 50°C (140°F) and cetyl alcohol, ARLACEL 165, BHT, parabens, lauroyl lysine, sesame oil, CROMOLLIENT, dimethicone, polydecene, almond oil, and oat oil were added. When the batch was niform it was neutralized with NaOH. The batch was cooled and glycerin VERSENE 100x1, fragrance and diazolidinyl urea were added at 45°C (113°F) or less. The batch was homogenized to form a creamy oil in water emulsion.

**Table 2**

| Sample 2 - Ingredients | Amount |
|---|---|
| Water | 82.82 |
| Glyceryl stearate | 1.25 |
| Stearic acid | 1.25 |
| Oleic acid | 1.25 |
| Polysorbate 61 | 1.20 |
| Isopropyl palmitate | 1.0 |
| Dimethicone | 1.0 |
| Sesame seed oil | 1.5 |
| CROMOLLIENT DP3A | 1.0 |
| Sorbitan stearate | 0.80 |
| Synthetic beeswax | 0.50 |
| Cetyl alcohol | 0.50 |
| Butylated hydroxytoluene | 0.02 |
| Stearyl alcohol | 0.50 |
| Benzyl alcohol | 0.30 |
| Methylparaben | 0.15 |
| Butylparaben | 0.05 |
| Propylparaben | 0.10 |
| Sodium Hydroxide (50% solution) | 0.26 |
| Perfume | 0.25 |
| Carbomer 934 | 0.30 |

The ingredients above are blended to form a creamy oil in water emulsion.

**Table 3**

| Sample 3 - Ingredients | Amount |
|---|---|
| Water | 70.64 |
| Colloidal oat flour | 1.00 |
| Sodium chloride | 0.01 |
| VARISOFT TA-100 | 5.00 |
| Glycerine 917 | 12.00 |
| Isopropyl palmitate | 1.0 |
| Dimethicone | 1.25 |
| Sesame seed oil | 4.0 |
| CROMOLLIENT DP3A | 2.0 |
| Benzyl alcohol | 0.60 |
| Cetyl alcohol | 2.50 |

The ingredients above are blended to form a creamy oil in water emulsion.

The materials for two water in oil emulsion formulations are listed in Tables 4 and 5:

**Table 4**

| Sample 4 - Ingredients | Amount |
|---|---|
| Water | 68.20 |
| Caprylic Capric Acid Triglyceride | 8.00 |
| Sodium chloride | 0.80 |
| Octylmethoxycinnamate | 5.00 |
| Avobenzone | 2.00 |
| Isopropyl stearate | 5.0 |
| Dimethicone copolyol | 5.00 |
| Phenoxyethanol/paraben | 1.0 |
| CROMOLLIENT DP3A | 5.0 |

The ingredients above are blended to form a creamy water in oil emulsion.

**Table 5**

| Sample 5 - Ingredients | Amount |
|---|---|
| Water | 79.30 |
| Sodium chloride | 0.70 |
| C12-15 alkyl benzoate | 9.0 |
| Dimethicone copolyol | 5.00 |
| Phenoxyethanol/paraben | 1.0 |
| CROMOLLIENT DP3A | 5.0 |

The ingredients above are blended to form a creamy water in oil emulsion.

The materials for two solution formulations are listed in Tables 6 and 7:

**Table 6**

| Sample 6 - Ingredients | Amount |
|---|---|
| Sesame oil | 36.00 |
| PPG 15 stearyl ether | 49.00 |
| PEG 40 sorbitan peroleate | 1.0 |
| Fragrance | 5.00 |
| CROMOLLIENT DP3A | 9.0 |

The ingredients above are blended to form a solution.

**Table 7**

| Sample 7 - Ingredients | Amount |
|---|---|
| Isopropyl palmitate | 36.00 |
| PPG 15 stearyl ether | 49.00 |
| PEG 40 sorbitan peroleate | 1.0 |
| Fragrance | 5.00 |
| CROMOLLIENT DP3A | 9.0 |

The ingredients above are blended to form a solution.

### Moisturization Test

The composition of sample 1 was tested to determine if it was useful as a moisturizer. *In vivo* spectra were measured using a Vector 22 FTIR Spectrometer (Bruker Analytical, GmbH, Hamburg, Germany) with a bifurcated fiber attached to it. A ZnSe crystal (diameter=3mm) was used as the Internal Reflectance Element (IRE) at the joint end of the fiber. Both the fiber and the crystal were from Remspec Corp. (Sturbridge, MA). The signal collected by the fiber was directed to a liquid nitrogen-cooled HgCdTe (MCT) detector (EG&G, Montgomeryville, PA). The incident light underwent two total reflections of 45° at the crystal/skin interface.

The spectra were routinely generated by co-addition of 128 interferograms at 4 cm⁻¹ resolution. The infrared peaks at ∼2850 and 2920 cm⁻¹ were originated from lipids (CH symmetric and asymmetric stretching mode) and the infrared peak at ∼3300 cm⁻¹ can be related to the hydration level of the stratum corneum. The intensities of these peaks were normalized to amide I peak (at ∼ 1650cm⁻¹) for each spectrum to avoid any variations induced by contact and these ratios were used as spectral parameters to evaluate lipids and water content on the skin surface.

Long lasting effect: The IR spectra were measured on the lower inner arms of six subjects before topical application of sample 1. The spectra were also measured at 3, 6, 8 and 24 hours after application for the same site. Spectral ratios were calculated and averaged for different subjects.

Occlusion: IR spectra were measured on the lower inner arms of two subjects before and after occlusion using sealwrap (Borden Packaging, North Andover, MA). The skin sites were occluded for 1 and 4 hours. IR spectra of the skin sites treated with the composition of sample 1 and a commercially available baby (mineral) oil were also measured before the application and 1 and 4 hours post application. The occlusion effects of the composition of sample 1 on skin hydration were evaluated by IR ratios and compared to baby (mineral) oil. The results of the water analysis for non-occlusive tests are shown in Table 8. The results of the occlusive tests for water in the skin and lipids on the skin are shown in Table 9.

**Table 8**

| Time (hours) | Relative Amount Of Water On Skin |
|---|---|
| 0 | 1 |
| 2 | 2.58 |
| 6 | 2.61 |
| 8 | 2.90 |

The data above demonstrate that the compositions of the present invention function well as moisturizers. The amount of water in the skin increased significantly after application of the composition of sample 1, and the water was retained over 8 hours.

**Table 9**

| Water In Skin | 0 Hours | 1 Hour | 2 Hours |
|---|---|---|---|
| Sample 1 | 0.28 | 0.62 | 0.85 |
| Mineral oil | 0.29 | 0.49 | 0.74 |
| Lipids On Skin | | | |
| Sample 1 | 0.21 | 1.04 | 1.03 |
| Mineral oil | 0.16 | 1.21 | 1.44 |

The data above demonstrate that the compositions of the present invention function similarly to commercially available mineral oil in terms of moisturization and retention on the surface of the skin.

### Consumer Test

Consumers (132 woman aged 25 to 54) were asked to apply the composition of Example 1 to their skin and given a questionnaire to evaluate the composition. Results are reported in Table 10.

**Table 10**

| Question | Percent Agreed |
|---|---|
| Definitely/probably would buy the product | 78 |
| Is a good moisturizer | 92 |
| Soothes dry, irritated skin | 90 |
| Relieves dry skin | 90 |
| Makes your skin feel silky | 89 |
| Absorbs quickly into skin | 92 |
| Does not leave skin feeling greasy/oily | 91 |

The data above demonstrate that the compositions of the present invention perform very well at moisturizing the skin and do not leave the skin feeling oily or greasy.

## Claims

1. A composition comprising:
an alkoxylated fatty alcohol acid ester of Formula I;
R₁-O-[CH₂-CH(CH₃)-O]_{y}-[(CH₂)₂-O]ₓ-C(O)-CH₂-B-CH₂-C(O)-[O-(CH₂)₂]ₓ-[O-CH(CH₃)-CH₂]-O-R₂ (I)
wherein:
B is alkylene of 0 to 10 carbon atoms which may be saturated or unsaturated, substituted or unsubstituted, or R₃-O-[CH₂-CH(CH₃)-O]_{y}-[(CH₂)₂-O]ₓ- C(O)-C(OH)<;and
R₁, R₂ and R₃ may be the same or different and each are fatty alcohols of 10 to 22 carbons which may be saturated or unsaturated, substituted or unsubstituted, x is 0 to 40 and y is 0 to 30 with the proviso that the total of all x^{s} and y^{s} is at least 6; and
at least one emollient, wherein the emollient is not mineral oil or petrolatum.

2. A composition according to claim 1 wherein the alkoxylated fatty alcohol acid ester is di-polypropylene glycol-3 myristyl ether adipate.

3. A composition according to claim 1 or claim 2 wherein the emollient is polypropylene glycol-15 stearyl ether, polypropylene glycol-10 cetyl ether, steareth-10, oleth-8, polypropylene glycol-4 lauryl ether, vitamin E acetate, polyethyleneglycol-7 glyceryl cocoate, lanolin, lauroyl lysine, almond oil, sesame seed oil, oat kernel oil, dimethylpolysiloxane or a combination thereof.

4. A composition according to claim 3 wherein the emollient is polypropylene glycol ("PPG")-15 stearyl ether, PPC-10 cetyl ether, PPG-4 lauryl ether, vitamin E acetate, polyethyleneglycol ("PEG")-7 glyceryl cocoate, lanolin, lauroyl lysine, almond oil, sesame see oil, oat kernel, rapeseed oil, macadamia nut oil, soybean oil, polydimethylsiloxane,isopropyl palmitate, isopropyl stearate, caprylic capric acid triglyceride, C12-15 alkyl benzoate or a combination thereof.

5. A composition according to any one of claims 1 to 4 further comprising at least one surfactant.

6. A composition according to claim 5 further comprising an emulsion stabilizer.

7. A composition according to claim 6 wherein the emulsion stabilizer is cetyl alcohol or an acrylate/C10-30 alkyl acrylate crosspolymer.

8. A composition according to any one of claims 5 to 7 wherein the composition is an oil in water emulsion.

9. A composition according to any one of claims 5 to 7 wherein the composition is a water in oil emulsion.

10. A composition according to any one of claims 1 to 9 for use in moisturizing skin by applying to the skin the composition in an amount effective to moisturize said skin.
